# EUROPEAN PATENT SPECIFICATION

(11) **EP 1 042 025 B1**
(45) Date of publication and mention of the grant of the patent: **03.06.2009**
(21) Application number: 98960364.2
(22) Date of filing: 24.11.1998
(51) Int. Cl.: A61M 15/00, A61M 16/00, B05D 7/14, B65D 83/04, B65D 83/06, B65D 85/42

(54) **DRY POWDER INHALER**
TROCKENPULVERINHALATOR
INHALATEUR DE POUDRE SECHE

(30) Priority: 02.12.1997 US 982320; 02.11.1998 US 184821
(43) Date of publication of application: 11.10.2000
(73) Proprietor: Valois SAS, 27110 Le Neubourg (FR)
(72) Inventor: JACKSON, Thomas, R., La Jolla, CA 92037 (US); DAVIES, Karen, San Diego, CA 92121 (US); CHEN, Jeff, Palo Alto, CA 94303 (US); LIGOTKE, Mike, San Diego, CA 92121 (US); CAMERON, Allan, Westlake Village, CA 91361 (US)
(74) Representative: CAPRI
(86) International application number: PCT/US1998/024914
(87) International publication number: WO 1999/027987

(56) References cited:
- US-A- 4 739 754
- US-A- 4 811 731
- US-A- 5 327 883
- US-S- D 384 283

## Description

The field of the invention is inhalers for delivering dry powder drugs to the lungs. Inhalers have long been used to deliver drugs into a patient's lungs. Typically, an inhaler provides a mixture of drugs and air or propellant gases. The mixture is delivered via the patient inhaling from a mouthpiece on the inhaler, for treatment of various conditions, for example, bronchial asthma. However, delivery of drugs via inhalation can be used for many other treatments, including those unrelated to lung condition.

Metered Dose Inhalers (MDIs) have been widely used for many years. MDI's typically dispense a single dose of a drug together with a propellant gas, with each actuation of the device. However, the propellant gases have been linked to destruction of the earth's ozone layer. In addition, with MDI's, the drug is generally released upon actuation of the device, regardless of whether the patient is properly inhaling during release. The patient may therefore not receive a complete dose unless the patient coordinates inhalation with actuation of the device. Achieving this coordination may be difficult for young children, or for patients with disabilities or under duress. Dry powder inhalers, on the other hand, do not have these disadvantages. Still, with dry powder inhalers, technical challenges remain in providing a reliable and simple to use device which can consistently deliver correct dosages of drugs.

One well known dry powder inhaler, the Diskhaler, described in U.S. Patent No. 4,627,432, uses individual drug doses sealed within blisters on a blister disk. A plunger pierces the blisters, to release each dose. The disk is advanced by a knob with each successive dose. The Spiros inhaler, described in U.S. Patent No. 5,622,166 is a dry powder inhaler which also uses a blister disk. Blisters are opened via shear tabs on the blister disk. The disk is advanced to provide the next dose by sliding the mouthpiece cover between open and closed positions. While these types of devices may have met with varying degrees of success, disadvantages remain in indexing or advancing a blister disk within an inhaler, with opening the blisters to access the drug contents, with reliably providing intended dosages, and in other areas. US-4,739,754 and US-4,811,731 also describe known inhalers.

Accordingly, it is an object of the invention to provide an improved dry powder inhaler.

To these ends, the invention provides an inhaler according to claim 1.

Advantageous embodiments are described in the dependent claims.

A drug dose carrier, such as a blister disk, is advantageously rotatably supported on or in a dry powder inhaler for pharmaceuticals. A slider is preferably attached to a housing or deck plate of the inhaler, and is movable between opened and closed positions. The slider may advantageously contain batteries or other electronic components electrically linked to components positioned in the main body of the inhaler via a flex circuit. Movement of the slider between the open and closed positions, in a preferred embodiment, opens a container on the carrier disk to release a pharmaceutical powder for inhalation. Preferably, this movement also advances the carrier, in preparation for delivery of a subsequent dose.

In a second aspect of the invention, a propeller spins within a mixing chamber and is turned by a turbine driven by the patient's inhalation.

In the drawings:
Fig. 1 is a perspective view of the inhaler of the invention with the slider in the closed or first position;
Fig. 2 is a perspective view thereof with the slider in the open or second position;
Figs. 3A and 3B are exploded top and bottom perspective views of the inhaler shown in Figs. 1 and 2;
Figs. 4A and 4B show enlarged perspective views of components of the inhaler of Figs. 3A and 3B;
Fig. 4C is a perspective view of the lifter shown in Fig. 4A;
Fig. 5 is a top perspective view of the deck plate shown in Figs. 3A and 3B;
Fig. 6 is a bottom perspective view thereof;
Fig. 7 is a section view thereof taken along a lateral centerline;
Fig. 8 is a perspective view of the flex circuit shown in Figs. 3A and 3B;
Figs. 9 and 10 are perspective views of the disk and cover shown in Figs. 3A, 3B and 4A;
Fig. 11 is a cross section view thereof taken through the anti-back up pawl shown in Fig. 9;
Fig. 12 is a central cross section thereof;
Fig. 13 is an exploded perspective view of a blister disk;
Fig. 14 is a top view thereof;
Fig. 15 is a section view thereof;
Fig. 16 is an exploded perspective view of another embodiment which is not covered by the invention having a turbine in place of an electric motor;
Fig. 17 is a perspective view of the inhaler shown in FIG. 16;
Fig. 18 is a perspective view thereof, also showing internal turbine details;
Fig. 19 is a schematic view showing air flow through the inhaler; and
Fig. 20 is an enlarged view of details shown in Fig. 19.

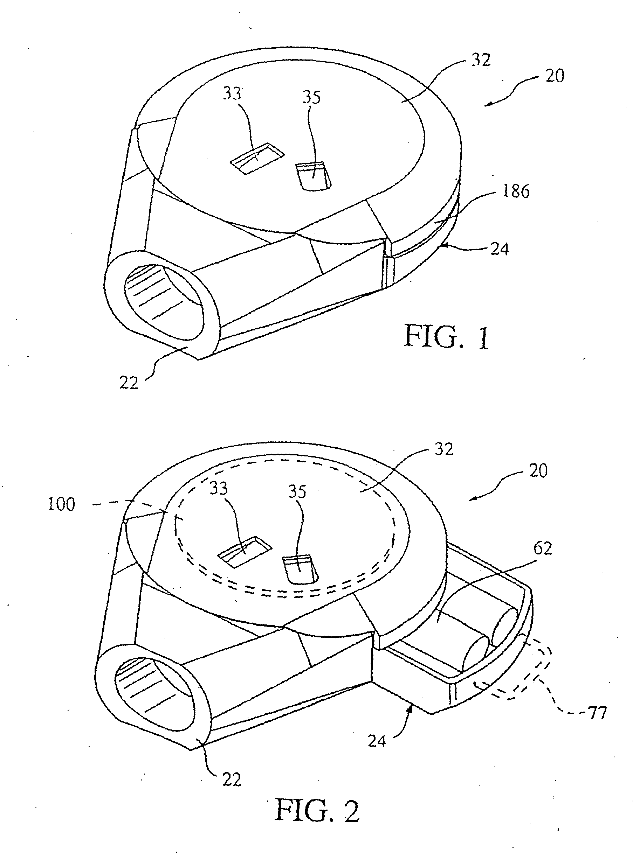

Turning now in detail to the drawings, the inhaler **20** includes a mouthpiece **22,** and a slider **24,** movable between a closed position, as shown in Fig. 1, and an open position as shown in Fig. 2. A handle **77** may be provided on the slider **24.**

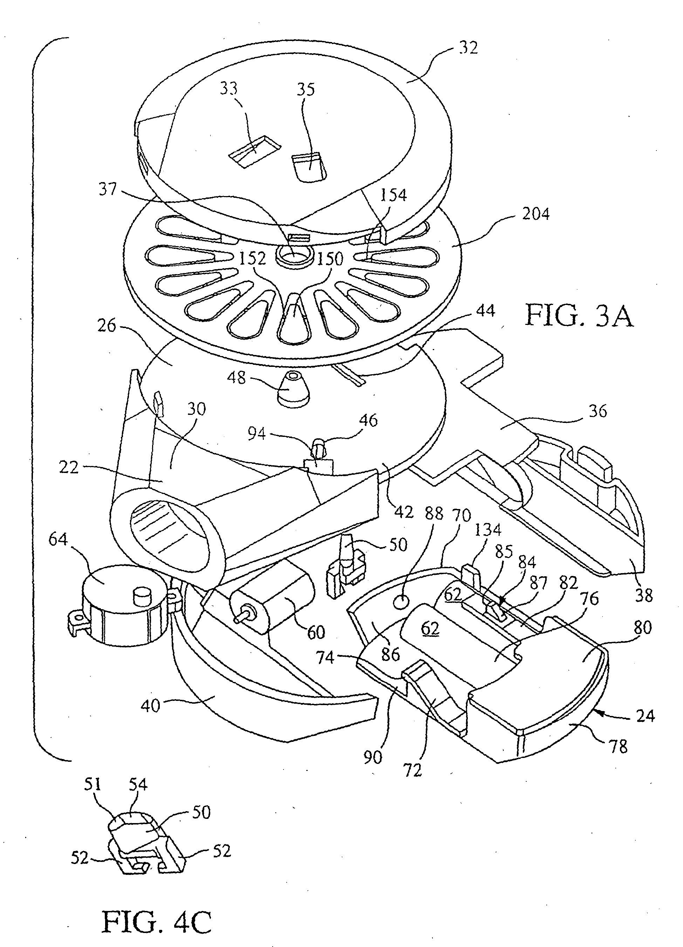

Turning to Figs. 3A, 3B and 5-7, a deck plate **42** has a flat top surface **26** and a flat bottom surface **28.** A spindle **48** extends upwardly from the top surface **26** of the deck plate **42.** A lifter slot **46** passes through the deck plate **42.** An advancing slot **44** extends through the deck plate parallel to the direction of motion of the slider **24.** A blister disk **34** used with the inhaler **20** includes a plurality of drug containing blisters **39** mounted on tabs **150**, as described, for example, in U.S. Patent No. 5,622,166.

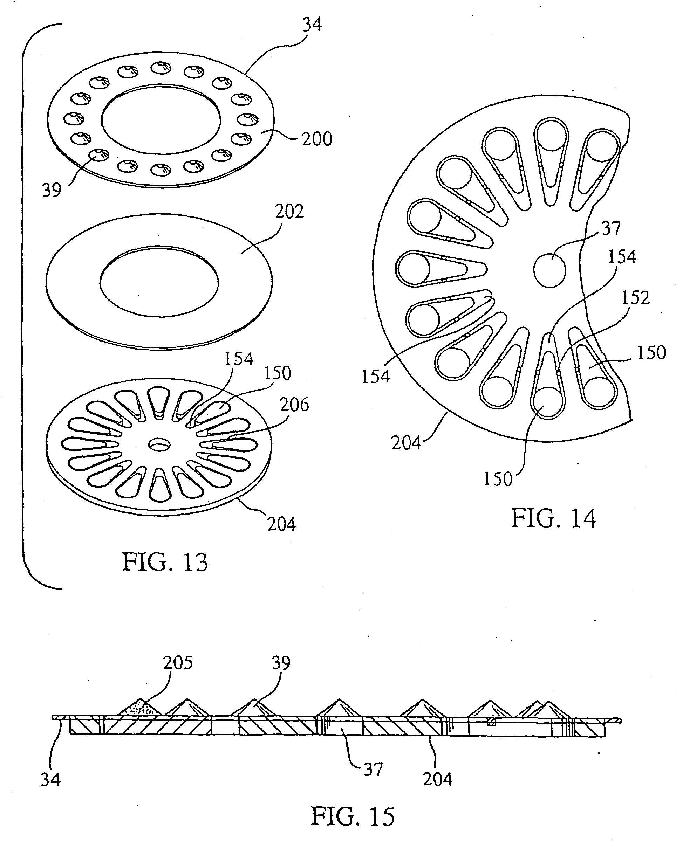

Turning momentarily to Figs. 13, 14 and 15, the blister disk **34** is made of a metal foil ring **200** having generally conical blisters **39** radially and equally spaced apart. The metal foil ring **200** and a seal ring **202** are adhered or bonded onto a carrier disk **204.** The disk **204** is preferably plastic. The carrier disk **204** has tabs **150** pivotably attached to the disk **204** via flex joints **152.** A blister **39** is aligned over each tab **150.** The flex joints **152** hold the tabs **150** into a flat, in-plane position, but allow the tabs **150** to pivot about the flex joints **152,** with nominal torque. As shown in Fig. 15, powdered drug **205** is contained within each blister **39.**

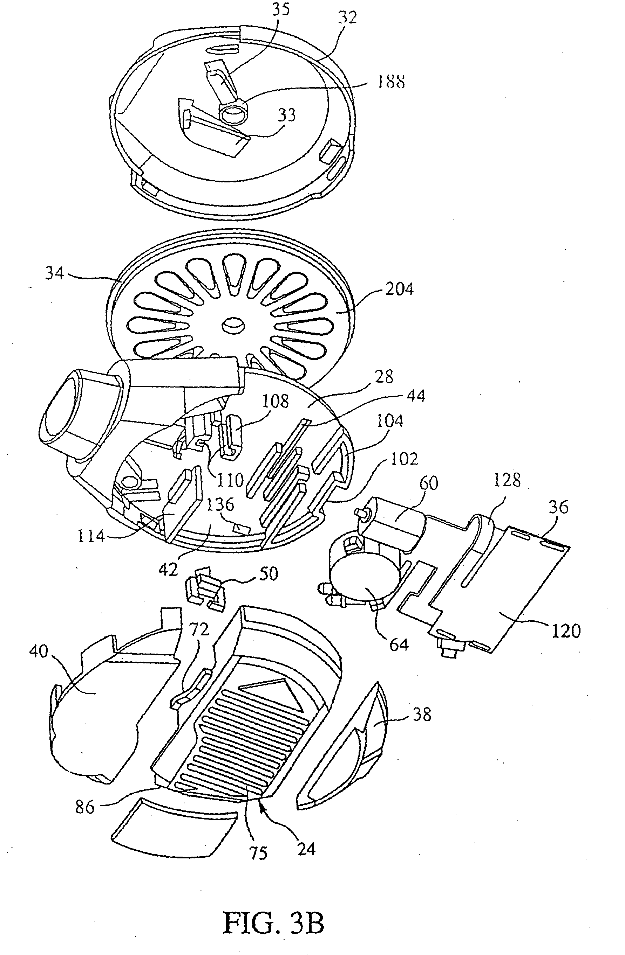

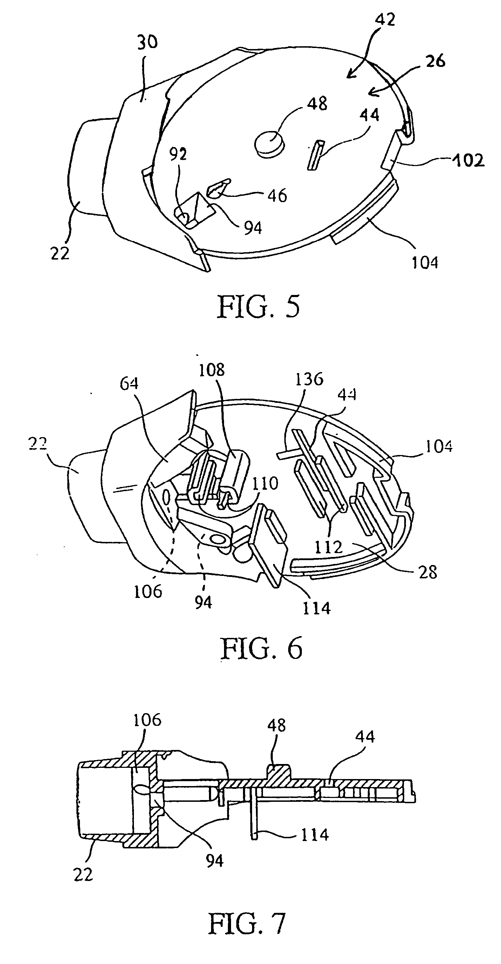

A cover **32** is attached over the top surface of a blister disk **34** to form a cover/disk assembly or unit **45.** The blister disk **34** has a central opening **37** so that it can be mounted on and rotate around the axis of the spindle **48** which is normal to the axis of motion of the slider. The cover **32** itself latches onto the deck and does not rotate. The cover **32** retains the blister disk onto the deck as the blister disk rotates. Referring specifically to Figs. 3B and 5-7, a back rim **104** extends downwardly from the deck plate **42,** opposite from the mouthpiece **22.** A recess **102** in the deck plate **42** and back rim **104** allows for the motion of a latch to lock the disk/cover assembly **45** onto the inhaler **20** when the cover blister disk assembly **45** is replaced by the user. A slider guide plate **114** extends downwardly from the bottom surface **28** of the deck plate **42.** Slider lever guides **112** similarly are positioned on the bottom surface **28** of the deck plate **42,** parallel to the advancing slot **44.** Lifter guides **108** forming leg slots **110** are also attached to the bottom surface **28** of the deck plate **42** under the lifter slot **46,** as best shown in Fig. 6. The leg slots **110** define the degree of freedom for the lifter motion.

A powder port **94** extends downwardly through the deck plate **42** and into an air passage **92** formed on the underside of the deck plate **42,** heading to an aerosolizing or mixing chamber **106.** The mouthpiece is advantageously removably attached to the deck plate **42.** The spindle **48,** back rim **104,** slide guide plate **114,** lever guides **112,** and lifter guides **108** are preferably integral with the deck plate.

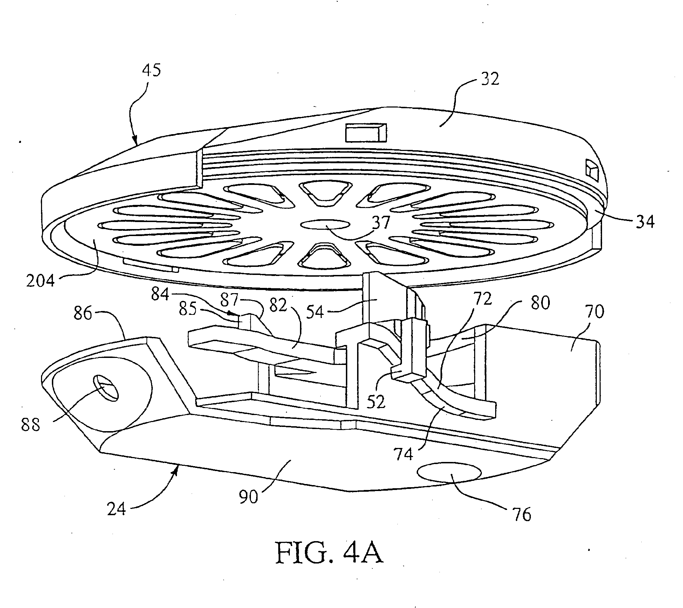

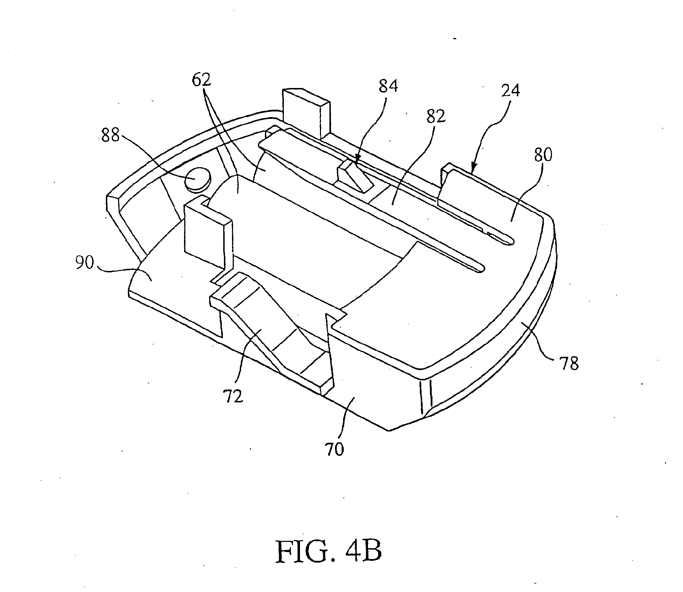

Referring back to Figs. 3A, 3B, 4A and 4B, the slider **24** includes a slider frame **70** attached to a bottom plate **90,** forming a battery compartment **76.** A lifter ramp **72** is attached or formed along one side of the slider bottom plate **90.** A lifter **50,** as shown in Figs. 4A, is slidably attached to the ramp **72.** The lifter **50** includes L-legs **52** for holding the lifter **50** onto lips **74** on the ramp **72** and for retracting the lifter **50** from its raised position. The lifter **50** has a flat top surface **51** and an angled surface **54.** The lifter ramp **72** passes under the lifter **50** as the slider **24** moves in and out. The lifter ramp **72** defines the cam profile which induces vertical motion in the lifter **50** as a result of the horizontal motion of the slider **24.**

Referring to Figs. 3A, 4A and 4B, a slider cover plate **80** is attached to the slider frame **70,** at the front or outside end **78** of the slider. A leg **82** on the slider cover plate **80** extends inwardly toward the rear end **86** of the slider **24.** A spring biased or resilient advancing finger **84** having a flat back surface **85** and an angled front surface **87** projects upwardly from the leg **82.** A battery check button extends through an opening **88** in the rear end **86** or bottom side **90** of the slider. As shown in Figs. 3A and 3B, a rear housing section **38** and a front housing section **40** are attached to the bottom surface of the deck plate **42,** on either side of the slider **24,** to enclose the inhaler and capture the slider in its guides, allowing only one degree of freedom. The front end **78** and rear end **86** of the slider are preferably shaped to match the contours of the inhaler.

A mechanical stop **134** on the slider engages a ledge **136** on the deck plate, to limit the outward sliding movement of the slider. Finger ridges or gripping features **75** may be provided on the bottom of the slider, as shown in Fig. 3B.

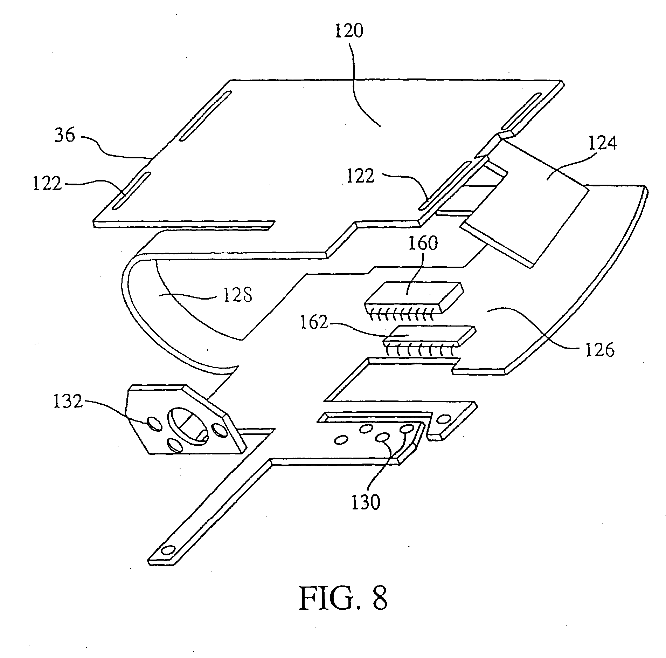

Referring to Figs. 3A, 3B and 8, a flex circuit **36** includes a battery plate **120** having battery slots **122.** A circuitry area **126** is electrically connected to the battery plate **120** by a flexible ribbon **128.** A switch **124** projects from the battery plate **120.** An LED plate **130** and a motor lead tab **132** are provided as part of the circuitry area **126.** A microprocessor **160** and memory chip **162** are provided on the circuitry area.

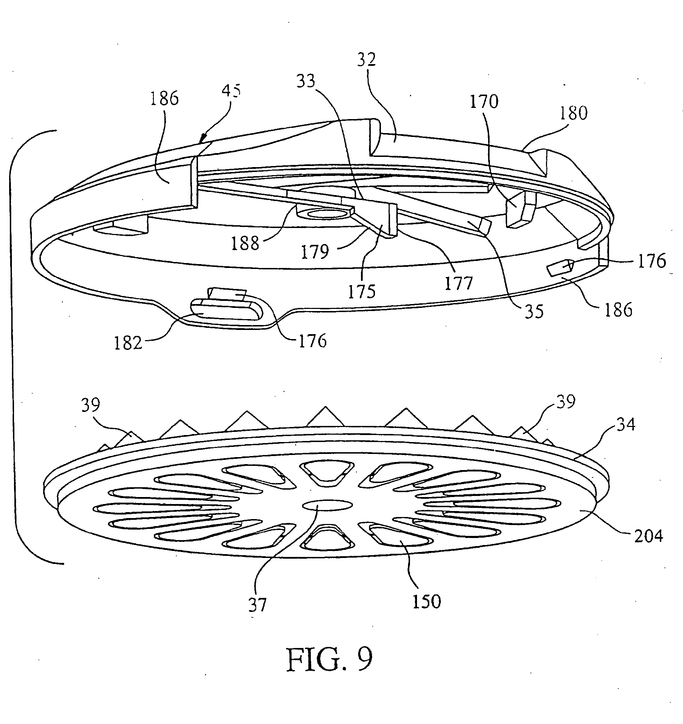

Turning to Figs. 9-12, a blister crushing rib **170** projects downwardly from the underside of the cover **32.** The crushing rib **170** is positioned so that when the cover/blister disk assembly **45** is assembled onto the inhaler **20,** the rib **170** aligns just behind the powder port **94.** A tab return spring **35** extends downwardly inside the cover **32.** The tab return spring **35** pushes downwardly on the inner end of the tab on the blister disk **34,** which is aligned over the lifter **50** and lifter slot **46.** An anti-backup pawl **33** also extends down inside the cover **32.** The pawl **33** has a foot **175** with a flat front surface **177** and an angled or ramp rear surface **179.**

Disk clips **176** having angled bottom facing surfaces are spaced apart around the inside of the cover **32,** on a cylindrical rim wall **186.** A front latch **180** and a rear latch **182** are provided for attaching the cover **32** to the inhaler **20**. Various latch designs may be used. Turning momentarily to Fig. 3A, the front latch fits into or engages a raised area 30 between the mouthpiece **22** and the disk assembly **45.**

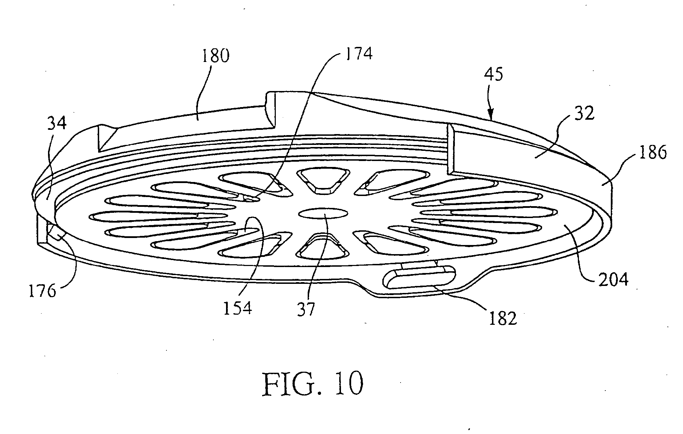

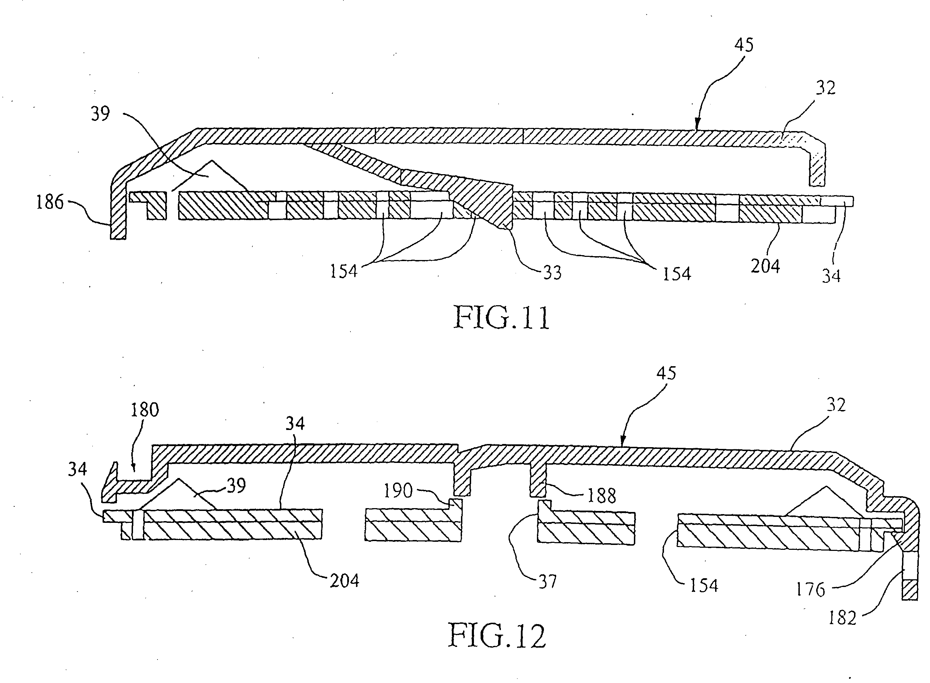

Referring to Figs. 10 and 12, a central hub **188** extends down from the center inside surface of the cover **32.** A blister disk **34** and a cover **32** are attached together to form an assembly **45** by aligning the central opening **37** or disk hub **190** of the disk with the hub **188** on the cover, and then pressing the disk into the cover. As this occurs, the clips **176** spring slightly apart, due to the natural resiliency of the cover material, and then snap back into place, thereby holding the disk and cover together. Once they are snapped together, they are substantially permanently yet rotatably attached to each other. As shown in Fig. 12, the disk **34** may have a perimeter recess **208,** such that the disk clips **176** clip onto a perimeter lip **210** around the outside of the disk.

As shown in Fig. 12, the disk hub **190** and cover hub **188** generally align but do not necessarily engage each other. The disk **34** floats somewhat in the cover. When installed, the disk centers itself on the spindle **48.**

Referring once again to Figs. 3A and 3B, an electric motor **60** is connected to batteries **62** in the slider **24** via the battery plate **120,** ribbon **128** and circuitry area **126** and motor lead tabs **132,** which have electrical conductors within them. A breath actuated switch or sensor **64** also attached to the deck plate **42** and flex circuit senses pressure at the mouthpiece **22,** and in response to sensed inhalation signals a microprocessor **160,** which switches on the motor **60.** The motor **60** spins an impeller within the mixing chamber **106,** as described in U.S. Patent No. 5,577,497.

A label **100,** shown in phantom line in Fig. 2, may be attached to the top of the cover **32,** to identify its contents, and to close off the openings where the pawl **33** and return spring **35** attach to the cover.

In use, the blister disk **34** is provided as an assembly **45** with the cover **32** attached. The cover **32** captures the blister disk **34** and secures them together. Hence, the cover **32** and blister disk **34** are handled as a unit or assembly by the patient. The cover protects the top and sides of the blister disk **34** from damage during handling. The patient attaches the cover/disk assembly **45** to the inhaler **20** via a bayonet, latch, rocker, or other attachment feature, such as the latches **180, 182.** The cover **32** allows the blister disk **34** to rotate on the spindle **48** while the cover itself is irrotatably snapped onto the deck plate. The cover is oriented so that the tab return spring **35** is located over the lifter slot.

With the cover/blister disk unit **45** secured to the inhaler **20** on top of the deck plate **42,** the inhaler **20** is ready for use. The patient pulls the slider **24** from the first or closed position shown in Fig. 1 to the second or open position shown in Fig. 2. Sliding movement of the slider is guided by the lever guides **112** and slider guide **114** and the covers **40** and **38.** Finger grips **75** or a handle **77** may be provided on the slider **24** to better facilitate pulling the slider out. The slider **24** moves out until the mechanical stop **134** on the slider frame **70** contacts the stop or ledge **136** on the deck plate **42.**

As the slider **24** is withdrawn, the lifter **50,** which is held in position by the lifter guides **108,** rides up on the lifter ramp **72.** This ramp lifting movement causes the angled top surface **54** of the lifter **50** to rise up and protrude through the lifter slot **46** in the deck plate **42.** The flat top surface **51** of the lifter **50** pushes against the underside of a tab **150** on the blister disk **34,** causing a blister positioned over the tab to shear open, as the tab pivots about flex joints **152** which attach the tab to the disk **34.** As the tab pivots, the angled surface **54** of the lifter engages the tab and continues to pivot it about the joints **152.** As the blister shears open, the powdered pharmaceutical **205** contained within the blister falls into the powder port **94** and air passage **92,** as described in U.S. Patent No. 5,622,166.

As the slider **24** is pulled out to the open position, the advancing finger **84** also moves down under the deck plate **42,** as it recedes out of the advance slot **44.**

The lifter **50** is restrained against lateral or longitudinal movement, by the engagement of the L-legs around the lips **74** on the lifter ramp **72** and by the lifter guides **108**. Accordingly, the lifter 50 can move only vertically up or down.

The patient then inhales on the mouthpiece **22.** The inhalation is sensed by the pressure switch **64** which turns on the motor **60.** The motor spins the impeller within the mixing chamber **106** creating an aerosol of air and powdered drug, as described in U.S. Patent No. 5,577,497. The inhalation draws substantially all of the drug from the powder port **94** and air passage into the mixing chamber **106,** for inhalation. When inhalation is complete, the pressure switch **64** shuts off the motor **60.** Inhalation preferably occurs when the slider is in the "out" position, when the blister is open and the inhaled air flow can draw any remaining drug out of the blister.

The patient next moves the slider **24** from the open position shown in Fig. 2 back to the closed position shown in Fig. 1. This movement is achieved by pushing on the slider front end **78.** As the slider moves back into the closed position, the lifter **50** is pulled down on the ramp **72.** The top surface **51** of the lifter **50** retracts to a position flush or below flush with the top surface of the deck plate **42.** The cam profile of the lifter ramp **72** is designed to allow the lifter **50** to return to its neutral (down) position before the blister disk is incrementally advanced. The tab return spring **35** exerts a downward force on the tab, to push the tab back into a horizontal orientation. The tab return spring also helps to keep the disk flat against the deck, by exerting a downward spring force on the disk. The cover hub **188** also exerts a downward force on the disk as well.

At the same time, the advancing finger **84** moves into the advance slot **44** and flexes or springs upwardly. The flat back surface **85** of the advancing finger protrudes upwardly through the advance slot **44** and extends into a tab slot **154** on the blister disk **34.** Continued closing movement of the slider **24** drives the advancing finger to rotate the disk **34.** The blister crushing rib **170** crushes down the conical point of the blister. This provides a visual indication through the transparent cover that the dose in that blister has been used and allows the patient to easily visually check the number of remaining doses on the disk. With the slider **24** completely moved back to the closed position, the advancing finger **84** turns the disk **34** through an angle which brings the next subsequent blister tab **150** into alignment with the lifer slot **46** and powder port **94.**

As the disk advances by one position, the foot **175** of the anti-backup pawl **174** rides up and out of the slot **154** and then drops back down into the next slot. The flat front surface **177** of the foot prevents reverse (clockwise when view from above) movement, while the ramp **179** on the back surface allows the foot and pawl to temporarily deflect up to allow the disk to advance in the forward direction.

The flex circuit **36** provides electrical connections between the batteries **62,** motor **60,** switch **64,** and other components, such as lamp indicators, microprocessor, memory chips, etc. The ribbon **128** on the flex circuit **36** allows the batteries and slider to remain electrically connected to the other fixed components, as the slider **24** is moved between open and closed positions. This motion can also be accomplished with flexible wires or cables.

The microprocessor controls the motor via the breath sensor; counts, doses delivered; signals low battery voltage; checks battery voltage, and controls LEDS which indicate use conditions of the inhaler.

The in and out movement of the slider is simple and easily achieved by almost all patients. It also provides for a reliable and compact design. Inadvertent actuation is also largely eliminated.

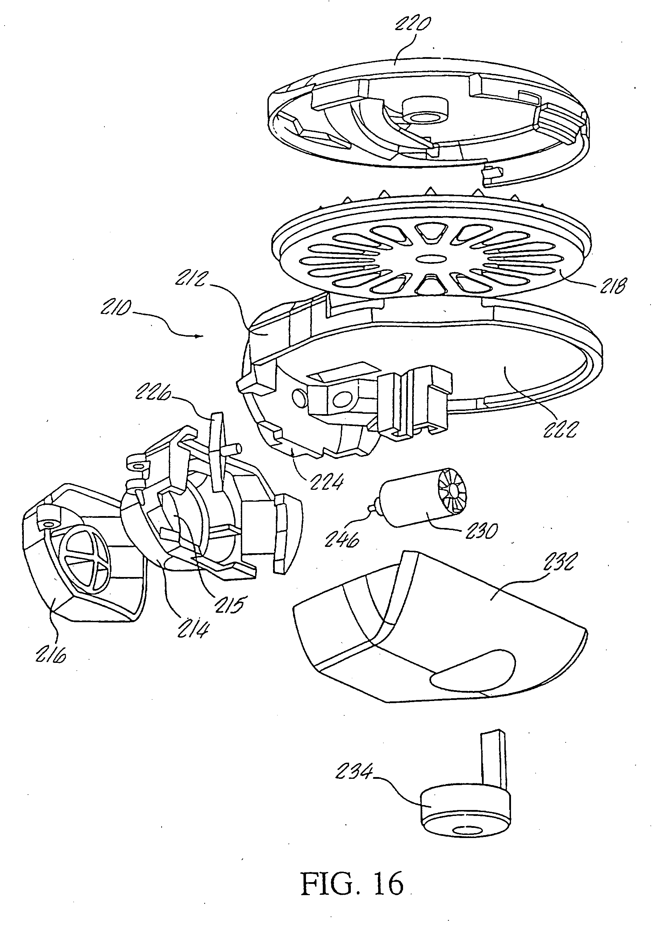

Turning now to Fig. 16, a turbine driven inhaler **210** has a mouthpiece **214** attached to a body **212.** A mouth piece cover **216** is attached to the mouth piece **214** via a hinge **215.** The mouth piece cover **216** can be pivoted open or removed during inhalation, or for cleaning.

A blister disk **218** having a transparent cover **220** is mounted over a disk plate **222** attached to the body **212.** An aerosolizing chamber **224** is formed in the front wall of the body **212.** These features may be similar to those shown in Fig. 4C.

A turbine **230** supported on the underside of the disk plate **222** has a turbine shaft **246** extending forwardly into the aerosolizing chamber **224.** A propeller **226** is mounted on the forward end of the turbine shaft **246** in the aerosolizing chamber **224.** A lower housing **232** encloses the bottom section of the disk plate **222.** A plunger **234** extends through the lower housing **232,** for opening blisters on the blister disk **218.** The body **212** and lower housing **232** form an inhaler housing **211.**

The design details and operation of the inhaler **210** are similar to the inhalers described in U.S. Patent Nos. 5,622,166. However, the inhaler **210** has no motor, batteries, switch or circuitry. Rather, the spinning propeller **226** is powered purely by the turbine **230.**

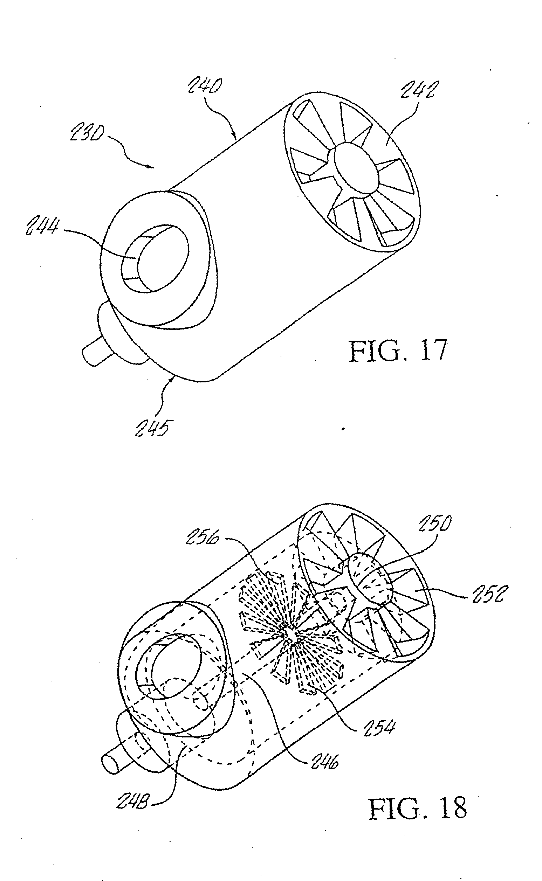

Referring to Fig. 17-20, the turbine **230** has a cylindrical turbine housing **240.** A stator **252** is joined to the turbine housing **240** near the turbine inlet **242.** A turbine outlet **244** is preferably positioned on one side of the cylindrical turbine housing **240,** at the outlet end **245** of the turbine **230.** The turbine shaft **246** is rotatably supported within the turbine housing **240** via a bushing **248** near the outlet end **245,** and via a needle bearing **250** near the inlet end **242.** A rotor **254** having pitched turbine blades **256** is attached and centered on the turbine shaft **246** adjacent to the stator **252.**

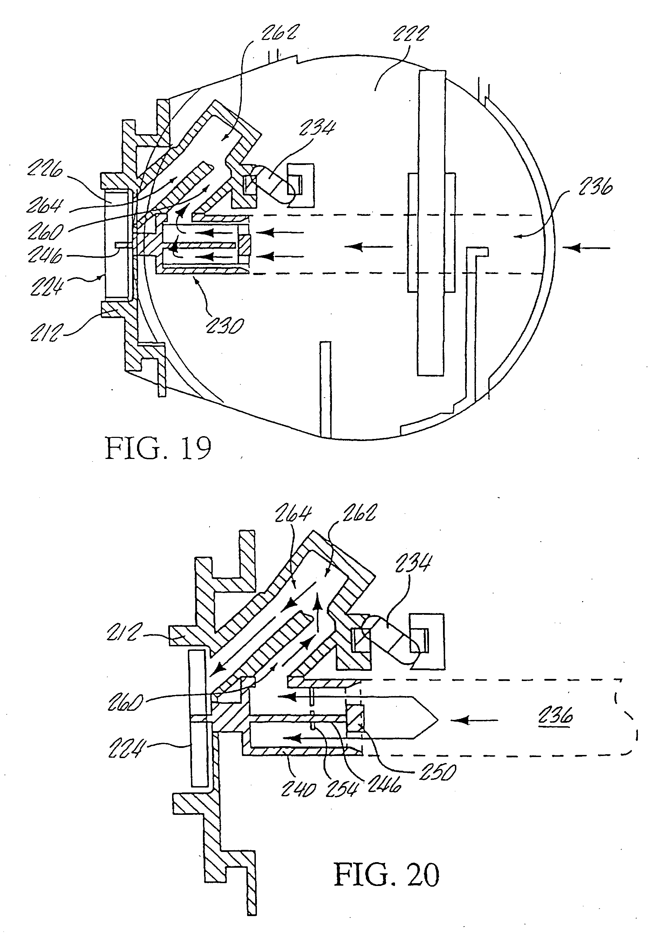

Turning to Figs. 19 and 20, an air inlet path **236** extends from the outside environment into the inhaler **210,** and to the inlet end **242** of the turbine **230.** A turbine outlet duct or path **260** runs from the turbine outlet **244** to a dump chamber **262** in the body **212** of the inhaler **210.** An aerosolizing chamber duct **264** extends from the dump chamber **262** to the aerosolizing chamber **224.** A chamber wall **215** in the mouth piece **214,** as shown in Fig. 16 forms the front wall of the aerosolizing chamber **224,** when the mouth piece **214** is attached on to the body **212.** Openings in the chamber wall **215** allow the drug/air mixture to flow from the aerosolizing chamber **224** through the mouth piece **214** to the patient.

In use, a dose of dry powered drug is delivered into the dump chamber **262** from the blister disk **218,** as described in U.S. Patent Nos. 5,622,166. The patient places the lips over the mouth piece **214** and inhales. Upon inhalation, ambient air flows through the inlet air path **236** to the inlet end **242** of the turbine **230.** The air flowing at right angles to the plane of the rotor **254** rapidly spins up the rotor and turbine shaft **246,** simultaneously rapidly spinning up the propeller 226 which is directly mounted on to the front end of the turbine shaft **246.** The rotor blades are pitched so that the air flow through the turbine, parallel to the axis of the turbine shaft, exerts torque, causing the rotor to spin. Air flows out of the turbine outlet 244 to the dump chamber **242.** Dry powder pharmaceutical particles are entrained in the air flow and carried through the aerosolizing chamber duct **264** into the aerosolizing chamber **224.** The particles are de-agglomerated and mixed with air in the aerosolizing chamber **224.** The air and particles pass out of the aerosolizing chamber **224** through openings in the chamber walls **215** and into the patients mouth, throat, and lungs.

Preferably, the turbine is designed to that the turbine shaft will spin at from 5,000-15,000 rpm with an inspiration flow rate of 20-40 liters per minute. Most preferably, the turbine 30 is designed so that it spins up to 10,000 rpm or greater, within 100 milliseconds, with an inspiration flow rate of about 30 liters per minute. The stator **252** may have fixed vanes to better direct air flow to the rotor **254.** Additional rotors **254** may optionally be added to the shaft **246.**

The air flow through the inhaler **210** is substantially sealed, so that all air inhaled by the patient passes through the inlet air path **236**, the turbine **230,** the turbine outlet duct **260,** the aerosolizing chamber duct **264,** the aerosolizing chamber **224,** and out through the mouth piece **214.** For embodiments not having a separate dump chamber, air flowing out of the turbine may go directly into the aerosolizing chamber. Alternatively, a fraction of the total airflow into the patients lungs may be either inletted or channeled through ducts in the mouthpiece or inhaler to help beneficially entrain, mix, or guide the particle laden air mixture.

The turbine **230** may advantageously be provided as a separate subassembly installed into the inhaler **210** during manufacture. As a result, various other components of the inhaler, not requiring the precision tolerances necessary in the turbine, can be manufactured and assembled separately. The turbine is compact, preferably having a housing diameter of 1-2 centimeters.

As shown in FIGs. 19 and 20, the dry powder does not flow through the turbine **230.** Rather, the turbine **230** is upstream from the powder. The turbine therefore avoids clogging, friction, or bearing failure from powder particles, as the turbine is upstream of the powder. Although the turbine **230** uses- the same air flow which entrains the powder, no balancing of air flow paths is required, and no coordination or timing of the spin-up of the turbine is needed, as the turbine automatically spins up upon inhalation.

The inhaler **210** consequently provides advantages of a motorized inhaler, without the need for a motor or batteries. If electronics are desired to provide an interface with the patient (for example, for dose counting, etc.) then very small batteries may be included to provide the typical low power requirements for such circuitry.

It may be desirable to allow the turbine enough time to reach a minimum acceptable rotary speed to de-agglomerate the drug, before the drug has passed through and out of the aerosolization chamber. One technique for this is to delay the introduction of the pharmaceutical mixture into the aerosolization chamber by sizing the length and diameter of the air path leading to the dump chamber. This allows the turbine time to reach the desired minimum rotational speed. As one example, if the outlet duct **260** is 1 cm diameter and 2.5 cm long, during the initial period of inhalation, at a flow rate of 5 liters per minute; the air takes 24 ms to reach the aerosolizing chamber. During that interval the turbine has accelerated up to a sufficient minimum speed.

Alternatively the inhaler may be inverted so that the air flowing through the turbine and hence 'over' the open well containing the blister has to reach a high enough velocity (i.e., **223** liters per minute, depending on how the local geometry is configured) to lift the particles out of the blister well due to Bernoulli's principal, rather than the particles just falling out of the well due to gravity even before the inhalation has begun. This could act as a passive method for regulating when the drug is introduced to the system based on the airflow rate.

In another embodiment intended to have the drug particles exposed to the spinning propeller in the aerosolization chamber is to place the restrictor holes, or outlet holes, near the center of the chamber rather than at the periphery. This would act like a centrifugal size filter, i.e. the larger particles would be forced to the periphery where the most aggressive de-agglomeration takes place until they are small enough to reach the more centralized outlet holes.

The turbine 230 may replace the motor in the inhalers shown in Figs. 1-7 or in the above referenced U.S. Patents.

## Claims

1. An inhaler for pharmaceuticals, comprising:
a housing (20);
a slider (24) slidably attached to the housing;
a ramp (72) and an advancing finger (84) attached to the slider;
**characterized in that** a lifter (50,52) is slidably attached to the housing and to the ramp, which lifter may move on said ramp to provide opening of a drug containing blister (39) as the slider slides out of the housing.

2. The inhaler of claim 1 further comprising a battery compartment (76) in the slider.

3. The inhaler of claim 2 further comprising a flex circuit (36) having a battery plate (120) at the battery compartment of the slider, connecting to a circuitry area (126) attached to the housing, via a flex ribbon (128).

4. The inhaler of claim 3 wherein the flex circuit includes electrical conductors extending through the battery plate, ribbon, and circuitry area, thereby providing continuous electrical contact between the circuitry area and the battery plate.

5. The inhaler of any one of the preceding claims wherein the advancing finger (84) on the slider advances a blister disk (34) including a plurality of blisters on the inhaler as the slider moves into the housing.

6. The inhaler of any one of the preceding claims further including a deck plate (42) attached to the housing, and having a top surface (26) and a bottom surface (28), a spindle (48) extending upwardly from the top surface for mounting a carrier disk (204), and a slider guide (114) extending downwardly from the bottom surface, for guiding the slider.

7. The inhaler of claim 6 further comprising an lifter guide (108) on the bottom surface of the deck plate, the lifter guide restraining the lifter in position, except along its path of motion.

8. The inhaler of claim 3 or 4 further comprising a mixing chamber (106) in the housing, a motor (60) in the housing, with the motor electrically connected to the flex circuit.

9. The inhaler of any one of the preceding claims further comprising a first mechanical stop and a second mechanical stop (134) on the housing for limiting travel of the slider into and out of the housing.

10. The inhaler of any one of the preceding claims further including:
a deck plate (42) in the housing, the deck plate including an advance slot (44) and a lifter slot (46), the advancing finger passing through the advance slot when the slider is in an in position, and with the lifter passing through the lifter slot, when the slider is in an out position.

11. The inhaler of any one of the preceding claims wherein the advancing finger (84) converts linear motion of the slider to an incremental pivoting motion of a blister disk, mounted on the housing.

12. The inhaler of any one of the preceding claims further including a cam profile on the ramp, with the lifter slidably attached to the ramp and following the cam profile as the slider moves into and out of the inhaler.

## Patentansprüche

1. Inhalator für Arzneimittel, aufweisend:
ein Gehäuse (20) ;
einen Schlitten (24), der an dem Gehäuse gleitbeweglich angebracht ist;
eine Rampe (72) und einen Vorrückfinger (84), der an dem Schlitten angebracht ist;
**dadurch gekennzeichnet, dass** eine Hubeinrichtung (50, 52) an dem Gehäuse und an der Rampe beweglich angebracht ist, wobei die Hubeinrichtung sich auf der Rampe bewegen kann, um das Öffnen eines Arzneimittel enthaltenden Blisters (39) bereit zu stellen, wenn der Schlitten sich aus dem Gehäuse heraus bewegt.

2. Inhalator nach Anspruch 1, außerdem aufweisend ein Batterieabteil (76) in dem Schlitten.

3. Inhalator nach Anspruch 2, außerdem aufweisend eine flexible Schaltung (36) mit einer Batterieplatte (120) an dem Batterieabteil des Schlittens zur Verbindung mit einem Schaltkreisbereich (126), der an dem Gehäuse angebracht ist, über ein flexibles Band (128).

4. Inhalator nach Anspruch 3, wobei die flexible Schaltung elektrische Leiter enthält, welche sich durch die Batterieplatte, das Band und den Schaltkreisbereich erstrecken und **dadurch** einen kontinuierlichen elektrischen Kontakt zwischen dem Schaltkreisbereich und der Batterieplatte bereitstellen.

5. Inhalator nach einem der vorangehenden Ansprüche, wobei der Vorrückfinger (84) auf dem Schlitten eine Blisterscheibe (34) vorrückt, welche mehrere Blister auf den Inhalator enthält, wenn der Schlitten sich in das Gehäuse bewegt.

6. Inhalator nach einem der vorangehenden Ansprüche, außerdem aufweisend eine an dem Gehäuse angebrachte Abdeckplatte (42), die eine Oberseite (26) und eine Unterseite (28), eine Spindel (48), die sich ausgehend von der Oberseite zur Anbringung einer Tragscheibe (204) erstreckt und eine Schlittenführung (114), die sich ausgehend von der Unterseite zum Führen des Schlittens nach unten erstreckt.

7. Inhalator nach Anspruch 6, außerdem aufweisend eine Hubeinrichtungsführung (108) auf der Unterseite der Abdeckplatte, wobei die Hubeinrichtungsführung die Hubeinrichtung bezüglich ihrer Stellung außer längs ihres Bewegungspfades zurückhält.

8. Inhalator nach Anspruch 3 oder 4, außerdem aufweisend eine Mischkammer (106) im Gehäuse, einen Motor (60) im Gehäuse, wobei der Motor mit der flexiblen Schaltung elektrisch verbunden ist.

9. Inhalator nach einem der vorangehenden Ansprüche, außerdem aufweisend einen ersten und einen zweiten mechanischen Anschlag (134) auf dem Gehäuse zum Begrenzen des Hubs des Schlittens in und aus dem Gehäuse heraus.

10. Inhalator nach einem der vorangehenden Ansprüche, außerdem aufweisend:
eine Abdeckplatte (42) in dem Gehäuse, wobei die Abdeckplatte einen Vorrückschlitz (44) und einen Hubeinrichtungsschlitz (46) aufweist, wobei der Vorrückfinger den Vorrückschlitz durchsetzt, wenn der Schlitten sich in einer innen liegenden Stellung befindet und wobei die Hubeinrichtung den Hubeinrichtungsschlitz durchsetzt, wenn der Schlitten sich in einer aussen liegenden Stellung befindet.

11. Inhalator nach einem der vorangehenden Ansprüche, wobei der Vorrückfinger (84) die lineare Bewegung des Schlittens in eine schrittweise Schwenkbewegung einer Blisterscheibe umsetzt, die auf dem Gehäuse angebracht ist.

12. Inhalator nach einem der vorangehenden Ansprüche, außerdem aufweisend ein Nockenprofil auf der Rampe, wobei der gleitbeweglich an der Rampe angebrachte Schlitten dem Nockenprofil folgt, wenn der Schlitten sich in den Inhalator hinein und aus diesem heraus bewegt.

## Revendications

1. Inhalateur pour produits pharmaceutiques, comprenant :
un boîtier (20) ;
un coulisseau (24) fixé de manière coulissante au boîtier ;
une rampe (72) et un doigt d'avancement (84) fixé au coulisseau ;
**caractérisé en ce qu'**un dispositif de levage (50, 52) est fixé de manière coulissante au boîtier et à la rampe, ledit dispositif de levage pouvant se déplacer sur ladite rampe pour réaliser l'ouverture d'un blister (39) contenant un médicament lorsque le coulisseau coulisse hors du boîtier.

2. Inhalateur selon la revendication 1 comprenant en outre un compartiment à batteries (76) dans la glissière.

3. Inhalateur selon la revendication 2, comprenant un circuit souple (36) ayant une plaque de batterie (120) au niveau du compartiment à batteries du coulisseau, se raccordant à une zone de circuits (126) fixée au boîtier, via un ruban souple (128).

4. Inhalateur selon la revendication 3, dans lequel le circuit souple comprend des conducteurs électriques s'étendant à travers la plaque de batterie, le ruban et la zone de circuits, fournissant ainsi le contact électrique continu entre la zone de circuits et la plaque de batterie.

5. Inhalateur selon l'une quelconque des revendications précédentes, dans lequel le doigt d'avancement (84) sur le coulisseau fait avancer sur l'inhalateur un disque de blister (34) comprenant une pluralité de blisters, lorsque le coulisseau se déplace dans le boîtier.

6. Inhalateur selon l'une quelconque des revendications précédentes comprenant en outre une plaque de pont (42) fixée au boîtier, et ayant une surface supérieure (26) et une surface inférieure (28), un axe (48) s'étendant vers le haut à partir de la surface supérieure pour monter un disque de support (204), et un guide de coulisseau (114) s'étendant vers le bas à partir de la surface inférieure, pour guider le coulisseau.

7. Inhalateur selon la revendication 6, comprenant en outre un guide de dispositif de levage (108) sur la surface inférieure de la plaque de pont, le guide de dispositif de levage retenant le dispositif de levage en position, excepté le long de sa trajectoire de mouvement.

8. Inhalateur selon la revendication 3 ou 4, comprenant en outre une chambre de mélange (106) dans le boîtier, un moteur (60) dans le boîtier, avec le moteur électriquement raccordé au circuit souple.

9. Inhalateur selon l'une quelconque des revendications précédentes, comprenant en outre une première butée mécanique et une seconde butée mécanique (134) sur le boîtier pour limiter le déplacement du coulisseau dans et hors du boîtier.

10. Inhalateur selon l'une quelconque des revendications précédentes, comprenant en outre :
une plaque de pont (42) dans le boîtier, la plaque de pont comprenant une fente d'avancement (44) et une fente de dispositif de levage (46), le doigt d'avancement passant à travers la fente d'avancement lorsque le coulisseau est dans une position entrée, et avec le dispositif de levage qui passe par le fente de dispositif de levage, lorsque le coulisseau est dans une position sortie.

11. Inhalateur selon l'une quelconque des revendications précédentes, dans lequel le doigt d'avancement (84) transforme le mouvement linéaire du coulisseau en mouvement pivotant incrémentiel d'un disque de blister, monté sur le boîtier.

12. Inhalateur selon l'une quelconque des revendications précédentes, comprenant en outre un profil de came sur la rampe, avec le dispositif de levage fixé de manière coulissante sur la rampe et suivant le profil de came lorsque le coulisseau se déplace dans et hors de l'inhalateur.
